(19) Europäisches Patentamt
European Patent Office
Office européen des brevets

(11) EP 2 630 113 B1

(12) EUROPEAN PATENT SPECIFICATION

(45) Date of publication and mention of the grant of the patent:
**11.12.2019 Bulletin 2019/50**

(21) Application number: **11775908.4**

(22) Date of filing: **12.10.2011**

(51) Int Cl.:
***C07C 53/128** (2006.01)* ***C07C 51/15** (2006.01)*
***C07D 303/16** (2006.01)* ***C08F 8/14** (2006.01)*
***C08G 63/91** (2006.01)* ***C09D 7/65** (2018.01)*

(86) International application number:
**PCT/EP2011/005105**

(87) International publication number:
**WO 2012/052126 (26.04.2012 Gazette 2012/17)**

# (54) GLYCIDYL ESTERS OF ALPHA, ALPHA BRANCHED NEONONANOIC ACIDS, SYNTHESIS AND USES

GLYCIDYLESTER VON ALFA, ALFA VERZWEIGTEN NEONONANSÄUREN, SYNTHESE UND VERWENDUNGEN

ESTERS DE GLYCIDYLE D'ACIDES ALPHA,ALPHA-NÉONONANOÏQUES RAMIFIÉS, LEUR SYNTHÈSE ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2010 EP 10015919**
**19.10.2010 EP 10013766**

(43) Date of publication of application:
**28.08.2013 Bulletin 2013/35**

(73) Proprietor: **Hexion Research Belgium SA**
**1348 Ottignies Louvain-la-Neuve (BE)**

(72) Inventors:
- **LE FEVERE DE TEN HOVE, Cédric**
  **B-1348 Ottignies Louvain-la-Neuve (BE)**
- **HEYMANS, Denis**
  **B-1348 Ottignies Louvain-la-Neuve (BE)**
- **STEINBRECHER, Christophe**
  **B-1348 Ottignies Louvain-la-Neuve (BE)**
- **KOTLEWSKA, Aleksandra**
  **NL-3196 KK Vondelingenplaat (NL)**
- **VAN'T SAND, Robert**
  **NL-3196 KK Vondelingenplaat (NL)**

(56) References cited:
**EP-A1- 1 033 360** **EP-A1- 1 281 700**
**EP-A1- 1 580 609**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 630 113 B1

## Description

The present invention relates to a manufacturing process for the preparation of α,α-branched alkane carboxylic acids providing glycidyl esters with an improved softness or hardness of the coatings derived thereof.

More in particular the invention relates to the preparation of aliphatic tertiary saturated carboxylic acids or α,α-branched alkane carboxylic acids, which contain 9 or 13 carbon atoms and which provide glycidyl esters with a branching level of the alkyl groups depending on the olefin feedstock used, and which is defined as below.

It is generally known from e.g. US 2,831,877, US 2,876,241, US 3,053,869, US 2,967,873 and US 3,061,621 that mixtures of α,α-branched alkane carboxylic acids can be produced, starting from mono-olefins, carbon monoxide and water, in the presence of a strong acid.

From e.g. H van Hoorn and G C Vegter, Dynamic modulus measurements as a tool in the development of paint base materials FATIPEC, Euro Continental Congress 9, p. 51-60 (1968); Rheol. Acta 10, p. 208-212 (1971); H van Hoorn, the influence of side group structure on the glass transition temperature of isomeric vinyl ester polymers, the relation between the final coating film properties and the isomer distribution in starting branched carboxylic acids, was known.

In such mixtures of α,α-branched alkane carboxylic acids, significant proportions of blocking β-methyl-branched carboxylic acid isomers were found, the properties of which have been found to antagonize the attractive properties of other α,α-branched saturated carboxylic acid constituents of said mixtures, when applied in the form of vinyl esters in the coating industry, requiring more and more so-called softer acid derivatives.

More in particular the conventionally produced α,α-branched carboxylic acid mixtures had been found to cause a too high hardness of the final coating of films, which was disadvantageous and therefore undesired for certain applications, due to the presence of significant proportions of blocking β-alkyl-branched isomers.

One of the more recent remedies has been disclosed in EP 1033360A1. The problem of providing better softening derivatives of α,α-branched acids, manufactured from alkenes, carbon monoxide and water and an acid catalyst was solved therein by a process, which actually comprised:

(a) oligomerisation of butene;
(b) separation of butene dimers and/or trimers from the oligomerisate;
(c) conversion of the butene dimers and/or trimers into carboxylic acids;
(d) conversion of the carboxylic acids into the corresponding vinyl esters showing attractive softening properties when mixed into other polymers or if used as comonomers in coatings.

An object of the above process are branched carboxylic acids, which are prepared from butene dimers obtainable by the OCTOL oligomerisation process and containing not more than 35 wt% of multi-branched olefins, such as dimethylhexene, and preferably at most 25 wt%. Moreover said carboxylic acids, having a significantly increased content of 2,2-dimethyl heptanoic acid and 2-ethyl,2-methyl hexanoic acid, and a decreased content of 2,3-dimethyl-2-ethyl pentanoic acid, could provide the presently required attractive properties of the corresponding vinyl esters, such as a Tg of -3°C at the lowest for the homopolymer of said Cg vinyl ester.

Another object of the disclosed process in EP 1 033 360 are branched $C_{13}$ carboxylic acids, which are prepared from butene trimers obtainable by the OCTOL oligomerisation process. Moreover said carboxylic acids, could provide the required attractive properties of the corresponding vinyl esters, such as a Tg of -13°C at the lowest for the homopolymer of said $C_{13}$ vinyl ester.

The EP1281700 is also about the manufacturing process for the preparation of alpha, alpha branched alkane carboxylic acids providing vinyl esters with improved softness. The softeness of the polymer is said to be correlated with the Tg of the vinylester-homopolymer, in this invention the specific acid catalyst $BF_3/H_3PO_4$ is leading to vinyl ester that provide such homopolymer.

The entire prior art is interested to provide soft monomers to be used as vinyl ester in latex formulations for example and to act as plasticizer.

An object of the present invention is to provide α,α-branched alkane carboxylic acids glycidyl ester derivatives in order to obtain attractive properties of coatings derived therefrom.

As a result of extensive research and experimentation a process giving the branched carboxylic acids aimed at, it has surprisingly been found, that the branching level of the glycidyl ester has a strong influence on the coating properties.

The present application is directed to a process for synthesis of glycidyl ester from butene oligomers, comprising:

(a) oligomerizing butenes or precursors of butene in presence of a catalyst characterized in that the olefin has a weight fraction of isobutene of at least 50 weight% on total alkene fraction of the mixture feed and that the subsequently mixture of neononanoic acid (C9 acids) derivative obtained by such a process is providing a mixture where the sum

of the concentration of the blocked and of the highly branched isomers is at least 50%, preferably above 60%, most preferably above 75%, orthat the olefin has a weight fraction of n-butene isomers of at least 50 weight% on total alkene fraction of the mixture feed, and the subsequently mixture of neononanoic acid (C9 acids) derivatives will provide a mixture where the sum of the concentration of concentration of blocked and highly branched isomers is maximum 55%, preferably below 40% and most preferably below 30%,
(b) converting butene oligomers of step (a) to carboxylic acids which are longer by one carbon atom, and
(c) converting the carboxylic acids to the corresponding glycidyl esters.

Accordingly, the invention relates to a manufacturing process for the preparation of $\alpha,\alpha$-branched alkane carboxylic acids, by reacting a mono-olefin or a precursor thereof, with carbon monoxide in the presence of a catalyst and water characterized in that the starting olefin is ethylene or ethylene oligomers, or butene or butene derivatives or butene precursors (such as alcohol), the most preferred are butene or butene oligomers. The industrial sources for butene are Raffinate I or Raffinate II or Raffinate III.

Raf I or any other mixture of alkane-alkene with a isobutene content of at least 50% weight on total alkene, are fractions used to produce a highly branched acid derivatives after dimerisation carboxylation and subsequently glycidation, the mixture of neo-acid (C9 acids) derivatives obtained by such a process and providing a mixture where the sum of the concentration of the blocked and of the highly branched isomers is at least 55%, preferably above 65%, most preferably above 75%.

If the olefin feed is based on Raf. II or Raf III or any mixture rich in n-butene isomers on the total olefins, the subsequently mixture of neo-acid (C9 or C13 acids) derivatives will provide a mixture where the concentration of blocked and highly branched isomers is maximum 55% preferably below 40% and most preferably below 30%.

We have discovered that well chosen blend of isomers of the glycidyl ester of neononanoic acids give different and unexpected performance in combination with some particular polymers such as polyester polyols. The isomers are described in Table 1 and illustrate in Figure 1.

From the prior art it is known that polyester resins based on the commercially available Cardura E10 often result in coatings with a low hardness and a poor drying speed. This low drying speed results in work time lost when the coating is applied e.g. on cars and other vehicles. One alternative to this drawback was suggested in the literature by using the pivalic glycidyl ester (EP 0 996 657), however this low molecular derivative is volatile.

There is also a need for glycidyl esters giving a lower viscosity to the derived polyesters or ether resins and epoxy systems but that are free of any safety risks.

We have found that the performance of the glycidyl ester derived from the branched acid is depending on the branching level of the alkyl groups $R^1$, $R^2$ and $R^3$, for example the neononanoic acid has 3, 4 or 5 methyl groups. Highly branched isomers are defined as isomers of neo-acids having at least 5 methyl groups.

Mixture of neononanoic acid providing a high hardness is a mixture where the sum of the concentration of the blocked and of the highly branched isomers is at least 50%, preferably above 60%, and most preferably above 75%.

Mixture of neononanoic acids providing soft polymers is a mixture where the concentration of blocked and highly branched isomers is maximum 55%, preferably below 40%, and most preferably below 30%.

The desired isomer distribution can be obtained by the selection of the correct starting olefin or precursors thereof, and also to a lesser extend by adjusting the Kock reaction conditions. The dimerisation of the olefin and/or the precussor thereof can be done for example according the method of EP1033360 and the subsequently carbonylation will provide the desired branched acid, which can be, for example glycidated according to PCT/EP2010/003334 or the US6433217.

The glycidyl esters so obtained can be used as reactive diluent for epoxy based formulations such as examplified in the technical brochure of Momentive(Product Bulletin: Cardura E10P The Unique Reactive Diluent MSC-521). Other uses of the glycidyl ester are the combinations with polyester polyols, or acrylic polyols, or polyether polyols. The combination with polyester polyols such as the one used in the car industry coating leads to a fast drying coating system with attractive coating properties.

The invention is further illustrated by the following examples, however without restricting its scope to these embodiments.

## Methods used

The isomer distribution of neo-acid can be determined by gas chromatography, using a flame ionization detector (FID). 0.5 ml sample is diluted in analytical grade dichloromethane and n-octanol may be used as internal standard. The conditions presented below result in the approximate retention times given in table 1. In that case n-Octanol has a retention time of approximately 8.21 minute.

The GC method has the following settings:

| | |
|---|---|
| Column: | CP Wax 58 CB (FFAP), 50 m x 0.25 mm, df = 0.2 μm |
| Oven program | : 150°C (1.5 min) - 3.5°C/min - 250°C (5 min) = 35 min |
| Carrier gas: | Helium |
| Flow : | 2.0 mL/min constant |
| Split flow : | 150 mL/min |
| Split ratio: | 1:75 |
| Injector temp | 250°C |
| Detector temp | 325°C |
| Injection volume: | 1 μL |

CP Wax 58 CB is a Gas chromatography column available from Agilent Technologies.

The isomers of neononanoic acid as illustrative example have the structure ($R^1$ $R^2$ $R^3$)-C-COOH where the three R groups are linear or branched alkyl groups having together a total of 7 carbon atoms.

The structures and the retention time, using the above method, of all theoretical possible neononanoic isomers are drawn in Figure 1 and listed in Table 1.

The isomers content is calculated from the relative peak area of the chromatogram obtained assuming that the response factors of all isomers are the same.

**Table 1:** Structure of all possible neononanoic isomers

| | R1 | R2 | R3 | Methyl groups | Blocking | Retention time [Minutes] |
|---|---|---|---|---|---|---|
| V901 | Methyl | Methyl | n-pentyl | 3 | No | 8.90 |
| V902 | Methyl | Methyl | 2-pentyl | 4 | Yes | 9.18 |
| V903 | Methyl | Methyl | 2-methyl butyl | 4 | No | 8.6 |
| V904 | Methyl | Methyl | 3-methyl butyl 1,1-dimethyl | 4 | No | 8.08 |
| V905 | Methyl | Methyl | propyl 1,2-dimethy | 5 | Yes | 10.21 |
| V906 | Methyl | Methyl | propyl 2,2-dimethyl | 5 | Yes | 9.57 |
| V907 | Methyl | Methyl | propyl | 5 | No | 8.26 |
| V908 | Methyl | Methyl | 3-pentyl | 4 | Yes | 9.45 |
| V909 | Methyl | Ethyl | n-butyl | 3 | No | 9.28 |
| V910 K1 | Methyl | Ethyl | s-butyl | 4 | Yes | 9.74 |
| V910 K2 | Methyl | Ethyl | s-butyl | 4 | Yes | 9.84 |
| V911 | Methyl | Ethyl | i-butyl | 4 | No | 8.71 |
| V912 | Methyl | Ethyl | t-butyl | 5 | Yes | 9.64 |
| V913 | Methyl | n-propyl | n-propyl | 3 | No | 8.96 |
| V914 | Methyl | n-propyl | i-propyl | 4 | Yes | 9.30 |
| V915 | Methyl | i-propyl | i-propyl | 5 | Yes | 9.74 |
| V916 | Ethyl | Ethyl | n-propyl | 3 | No | 9.44 |
| V917 | Ethyl | Ethyl | i-propyl | 4 | Yes | 10.00 |

**Figure 1:** Structure of all possible neononanoic isomers

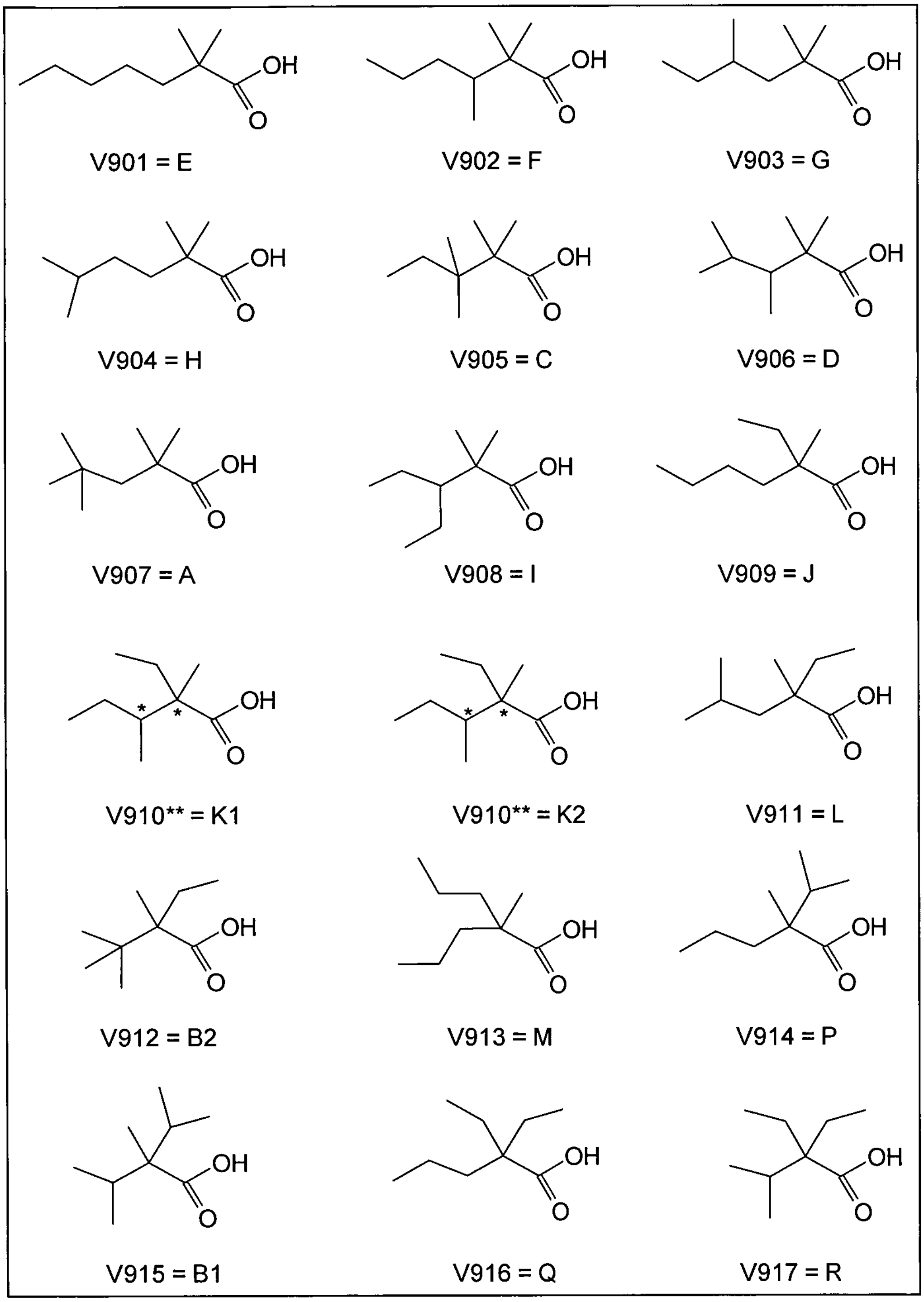

V901 = E
V902 = F
V903 = G
V904 = H
V905 = C
V906 = D
V907 = A
V908 = I
V909 = J
V910** = K1
V910** = K2
V911 = L
V912 = B2
V913 = M
V914 = P
V915 = B1
V916 = Q
V917 = R

**Blocking isomers**

Whereas the carbon atom in alpha position of the carboxylic acid is always a tertiary carbon atom, the carbon atom(s) in β position can either be primary, secondary or tertiary. Neononanoic acids (V9) with a secondary or a tertiary carbon atoms in the β position are defined as blocking isomers (Figures 2 and 3).

Fig 2

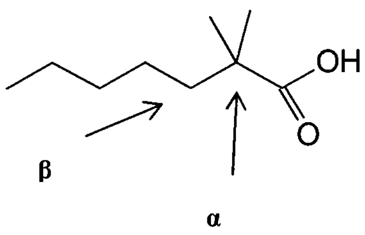

Fig 3

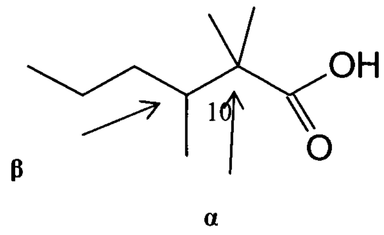

Figure 2: Example of a Non-blocked V9 Structure
Figure 3: Example of a Blocked V9 Structure

**Methods for the characterization of the resins**

The molecular weights of the resins are measured with gel permeation chromatography (Perkin Elmer/ Water) in THF solution using polystyrene standards. Viscosity of the resins are measured with Brookfield viscometer (LVDV-I) at indicated temperature. Solids content are calculated with a function (Ww-Wd) / Ww × 100%. Here Ww is the weight of a wet sample, Wd is the weight of the sample after dried in an oven at a temperature 110 °C for 1 hour.

**Methods for the Characterization of the Clear Coats**

Pot-life

Pot-life is determined by observing the elapsed time for doubling of the initial viscosity at room temperature, usually 24.0 ± 0.5 °C. The initial viscosity of the clear coat is defined at 44-46 mPa.s for Part 1 and 93-108 mPa.s for Part 3 measured with Brookfield viscometer.

Application of clearcoat

Q-panels are used as substrates. Then the panels are cleaned by a fast evaporating solvent methyl ethyl ketone or acetone. For Part 1 the clearcoat is spray-applied on Q-panels covered with basecoat; for Parts 2 & 3 the clearcoat is barcoated directly on Q-panels.

Dust free time

The dust free time (DFT) of clear coat is evaluated by vertically dropping a cotton wool ball on a flat substrate from a defined distance. When the cotton ball contacts with the substrate, the substrate is immediately turned over. The dust free time is defined as the time interval at which the cotton wool ball no longer adhered to the substrate.

Hardness development

Hardness development is followed using pendulum hardness tester with Koenig method.

**Chemicals used:**

**Curing agents**

**HDI:** 1,6-hexamethylene diisocyanate trimer, Desmodur N3390 BA from Bayer Material Science or Tolonate HDT LV2 from Perstorp
**Leveling agent:** 'BYK 10 wt%' which is BYK-331 diluted at 10% in butyl acetate
**Catalyst:** 'DBTDL 1 wt%' which is Dibutyl Tin Dilaurate diluted at 1wt% in butyl acetate
**Thinner:**

**A:** is a mixture of Xylene 50wt%, Toluene 30wt%, ShellsolA 10wt%,2-Ethoxyethylacetate 10wt%.

**B:** is butyl acetate

**Monopentaerythritol** : available from Sigma - Aldrich
**Methylhexahydrophtalic anhydride:** available from Sigma - Aldrich
**acrylic acid :** available from Sigma - Aldrich
**hydroxyethyl methacrylate:** available from Sigma - Aldrich
**styrene** : available from Sigma - Aldrich
**methyl methacrylate** : available from Sigma - Aldrich
**butyl acrylate** : available from Sigma - Aldrich
**tert-Butyl peroxy-3,5,5-trimethylhexanoate:** available from Akzo Nobel
**Di-tert-butyl peroxide** : Luperox Di from Arkema
**Cardura™ E10:** available from Hexion Specialty Chemicals
**GE9S:** glycidyl ester of C9 neo-acids obtained by dimerisation of n-butene, or Raf. II or Raf. III (leading to a mixture where the concentration of blocked and highly branched isomers is maximum 55% preferably below 40%) in presence of CO, a catalyst, water and subsequently reacted with epichlorohydrin
**GE9H:** glycidyl ester of C9 neo-acids obtained by dimerisation of iso-butene, or Raf. I (leading to a mixture where the concentration of the blocked and of the highly branched isomers is at least 55%, preferably above 65%) in presence of CO, a catalyst, water and subsequently reacted with epichlorohydrin
**GE5:** glycidyl ester of pivalic acid obtained by reaction of the acid with epichlorhydrin.

**EXAMPLES**

Example 1

Monopentaerythritol / Methylhexahydrophtalic anhydride / GE9S (1/3/3 molar ratio) = CE-GE9S

80.4 g amount of butylacetate, 68.3 g of monopentaerythritol, 258.2 g of methylhexahydrophthalic anhydride are loaded in a glass reactor and heated to reflux until complete dissolution. Afterwards, the temperature is decreased down to 120°C and 333.0 g of GE9S are added over about one hour. The cooking is pursued at 120°C for the time needed to decrease epoxy group content and acid value down to an acid value below 15 mg KOH/g. Then, further 82.4 g of butylacetate are added. Test results are indicated in table 2.

Example 2a

Monopentaerythritol / Methylhexahydrophtalic anhydride / GE9H (1/3/3 molar ratio) = CE-GE9H a

80.4 g amount of butylacetate, 68.3 g of monopentaerythritol, 258.2 g of methylhexahydrophthalic anhydride are loaded in a glass reactor and heated to reflux until complete dissolution. Afterwards, the temperature is decreased down to 120°C and 337.1 g of GE9H are added over about one hour. The cooking is pursued at 120°C for the time needed to decrease epoxy group content and acid value down to an acid value below 15 mg KOH/g. Then, further 83.4 g of butylacetate are added.

Example 2b

Monopentaerythritol / Methylhexahydrophtalic anhydride / GE9H (1/3/3 molar ratio) = CE-GE9Hb

CE-GE9Hb is a duplication of Example 2a performed in very close experimental conditions.

Comparative example 1a according to EP 0996657 Monopentaerythritol / Methylhexahydrophtalic anhydride / GE5 (1/3/3 molar ratio) CE-GE5a

71.3 g amount of butylacetate, 60.5 g of monopentaerythritol, 228.90 g of methylhexahydrophthalic anhydride are loaded in a glass reactor and heated to reflux until complete dissolution. Afterwards, the temperature is decreased down to 120°C and 214.3 g of GE5 are added over about one hour. The cooking is pursued at 120°C for the time needed to decrease epoxy group content and acid value down to an acid value below 15 mg KOH/g. Then, further 52.1 g of butylacetate are added.

Comparative example 1b according to EP 0996657 Monopentaerythritol / Methylhexahydrophtalic anhydride / GE5 (1/3/3 molar ratio) CE-GE5b

CE-GE5b is a duplication of comparative example 1a performed in very close experimental conditions except for the higher amount of butylacetate added at the end of the reaction.

**Table 2:** Polyesters characterization

| Polyester resin | SC(%) | Mw (Da) | Mn (Da) | Mw/Mn (PDI) | Viscosity (cP) |
|---|---|---|---|---|---|
| CE-GE9S | 78.6 | 974 | 919 | 1.06 | 2450 (25.9°C) |
| CE-GE9Ha | 80.0 | 921 | 877 | 1.05 | 6220 (25.9°C) |
| CE-GE9Hb | 80.0 | 1014 | 975 | 1.04 | 11740 (21.6°C) |
| CE-GE5a | 79.3 | 914 | 886 | 1.03 | 5080 (26.0°C) |
| CE-GE5b | 68.3 | 1177 | 1122 | 1.05 | 102.3 (22.0°C) |
| SC : solids content | | | | | |

Example 3

Acrylic resin synthesis

Cardura™ E10 based acrylic polyol resin: Acryl-CE(10)
105.0 g amount of CE10 (Cardura™ E10-glycidyl ester of Versatic acid) and 131.6 g of Shellsol A are loaded in a glass reactor and heated up to 157.5°C. Then, a mixture of monomers (37.4g acrylic acid, 107.9g hydroxyethyl methacrylate, 180.0g styrene, 100.2 g butyl acrylate, 69.6g methyl methacrylate) and initiator (12.0 g Di-tert-butyl peroxide) is fed into the reactor at a constant rate in 5 hours. Then post cooking started: a mixture of 6.0 g Di-tert-butyl peroxide and 18.0 g n-butyl acetate is fed into the reactor at a constant rate in 0.5 hour, then temperature maintained at about 157.5°C for a further 0.5 hour. Finally, 183.2g of n-butyl acetate is added under stirring to achieve a polyol resin with the target solids content. Test results are indicated in table 3.

**Table 3:** Acryl-CE(10) characterization

| Acryl- | SC (%) - measured | Mw (Da) | Mn (Da) | Mw/Mn (PDI) |
|---|---|---|---|---|
| CE(10) | 65.2 | 5094 | 2629 | 1.94 |

Three types of formulations have been prepared:

Blend Acryl-CE(10) blend with CE-GEx polyester with Desmodur as hardener (**Part 1**)
CE-GEx polyester alone with Tolonate HDT LV2 as hardener (0.03 wt% DBTDL)(**Part 2**)
CE-GEx polyester alone with Tolonate HDT LV2 as hardener (0.09wt% DBTDL) (**Part 3**)

**Part 1: CE-GEx polyesters blend with Acryl-CE(10) Formulation**

**Table 4:** Clear coats, formulations

| CE-GEx | Binder 1 (g) | Binder 2 (g) | HDI (g) | BYK 10 wt% (g) | DBTDL 1wt% (g) | Thinner A (g) |
|---|---|---|---|---|---|---|
| **GE9Hb** | 71.6 | 16.9 | 31.2 | 0.63 | 1.39 | 86.33 |
| **GE5b** | 79.1 | 12.4 | 31.2 | 0.63 | 1.39 | 89.30 |
| Binder 1: Acryl-CE(10)<br>Binder 2: CE-GEx polyesters | | | | | | |

(Part 1 - CE-GEx polyesters blend with Acryl-CE(10))

**Part 2 - CE-GEx polyesters alone,** no Acryl-CE(10) **Formulation** (0.03wt% DBTDL)

**Table 5:** Clear coats, formulations

| CE-GEx | Binder 2 (g) | HDI (g) | BYK 10wt% (g) | DBTDL 1wt% (g) | Thinner B (g) |
|---|---|---|---|---|---|
| **GE9S** | 80.0 | 36.56 | 0.72 | 3.15 | 89.75 |
| **GE9H a** | 80.4 | 37.27 | 0.73 | 3.20 | 87.83 |
| **GE5 a** | 79. 9 | 43.18 | 0.76 | 3.36 | 94.82 |

(Part 2 - CE-GEx polyesters alone)

**Part 3** - **CE-GEx polyesters alone,** no Acryl-CE(10) **Formulation** (0.09wt% DBTDL)

**Table 6:** Clear coats, formulations

| CE-GEx | Binder 2 (g) | HDI (g) | BYK 10wt% (g) | DBTDL 1wt% (g) | Thinner B (g) |
|---|---|---|---|---|---|
| **GE9H a** | 60.0 | 28.10 | 0.54 | 7.18 | 15.40 |
| **GE5 a** | 59.8 | 32.54 | 0.57 | 7.57 | 17.79 |

(Part 3 - CE-GEx polyesters alone)

**Characterization of the Clear Coats**

The clearcoat formulations are barcoat applied on degreased Q-panel for Parts 2 & 3; sprayed for the Part 1 on Q-panel with a basecoat. The panels are dried at room temperature, optionally with a preliminary stoving at 60°C for 30 min.

**Part 1** - **CE-GEx polyesters blend with Acryl-CE(10)** / **Room temperature curing**

**Table 7:** Clear coats, performances

| **CE-GEx** | **SC** (%) | **Potlife** (h) | **Drying conditions** | **DFT** (min)<br>*Cotton Balls* |
|---|---|---|---|---|
| GE9Hb | 47.1 | 4.5 | RT | 15 |

(continued)

| **CE-GEx** | **SC** (%) | **Potlife** (h) | **Drying conditions** | **DFT** (min) *Cotton Balls* |
|---|---|---|---|---|
| GE5b | 46.2 | 4.0 | RT | 19 |
| SC: solids content, RT: room temperature | | | | |

(Part 1 - CE-GEx polyesters blend with Acryl-CE(10)

**Part 2 - CE-GEx polyesters alone,** no Acryl-CE(10) / **Room temperature curing and room temperature drying after stoving**

**Table 8:** Clear coats, performances

| **CE-GEx** | **SC** (%) | **Drying conditions** | **DFT** (min) *Cotton Balls* | **Koenig Hardness** (s) | | |
|---|---|---|---|---|---|---|
| | | | | **6h** | **24h** | **7d** |
| GE9S | 48.4 | RT | 223 | 3 | 17 | 159 |
| GE9H a | 49.2 | RT | 91 | 3 | 36 | 212 |
| GE5 a | 49.5 | RT | 114 | 1 | 29 | 216 |
| | | | | | | |
| GE9S | 48.4 | Stoving 30 min/60°C | Dust free out of oven | 4 | 44 | 174 |
| GE9H a | 49.2 | Stoving 30 min/60°C | Dust free out of oven | 10 | 55 | 211 |
| GE5 a | 49.5 | Stoving 30 min/60°C | Dust free out of oven | 6 | 49 | 216 |

(Part 2 - CE-GEx polyesters alone, **no Acryl-CE(10)**

**Part 3** - **CE-GEx polyesters alone,** no Acryl-CE(10) / **Room temperature curing and room temperature drying after stoving** (0.09wt% DBTDL)

**Table 9:** Clear coats, performances

| **CE-GEx** | **SC** (%) | **Potlife** (min) | **Drying conditions** | **DFT** (min) *Cotton Balls* | **Koenig Hardness** (s) | | |
|---|---|---|---|---|---|---|---|
| | | | | | **6h** | **24h** | **7d** |
| GE9H a | 69.8 | 42.4 | RT | 47 | 3 | 66 | 193 |
| GE5 a | 68.5 | 61.3 | RT | 73 | 3 | 55 | 191 |
| | | | | | | | |
| GE9H a | 69.8 | 42.4 | Stoving 30 min/60°C | Dust free out of oven | 29 | 102 | 210 |
| GE5 a | 68.5 | 61.3 | Stoving 30 min/60°C | Dust free out of oven | 12 | 69 | 205 |

(Part 3- CE-GEx polyesters alone, no Acryl-CE(10))

**Observations**

**Part 1**

The potlife is about the same, the dust free time is shorter for GE9Hb vs. GE5b.

**Part 2**

The 24h hardness order GE9H, GE5 and GE9S and the dust free time at room temperature is the best for GE9H.

**Part 3**

The hardness development is the best for GE9H at room temperature and heat cure, the dust free time at room temperature is quicker for GE9H than for GE5; and with a volatile organic content of 300 g/l.

Example 4

Polyether resin

The following constituents were charged to a reaction vessel equipped with a stirrer, a thermometer and a condenser: 134 grams of di-Trimethylol propane (DTMP), 900 grams of glycidyl neononanoate, GE9 H, 135.5 grams of n-butylacetate (BAC) and 2.5 grams of Tin 2 Octoate. The mixture was heated to its reflux temperature of about 180° C. for about 4 hours till the glycidyl neononaoate was converted to an epoxy group content of less than 0.12 mg/g. After cooling down the polyether had a solids content of about 88%.

Example 5

Preparation for Vacuum infusion of composite structures

A resin for vacuum infusion of large structures such as yacht and wind turbines was prepared by mixing 27.7 part by weight of curing agent blend and 100 part of epoxy resins blend described here:

Epoxy resins blend: 850 part by weight Epikote 828 and 150 part of glycidyl neononanoate, GE9 H.
Curing Agent blend: 650 part by weight of Jeffamine D230 and 350 part by weight of Isophorone diamine (IPDA).

Jeffamine D230 is a polyoxyalkyleneamines available from Huntsman Corporation. Epikote 828 is an epoxy resin available from Momentive Specialty Chemicals

Example 6

Example of trowellable floor and patching compound

The ingredients presented in the table below were mixed for the preparation of a trowellable flooring compound

| BASE COMPONENT | Weight (parts) | Volume (parts) | Supplier |
|---|---|---|---|
| EPIKOTE 828LVEL | 63.2 | 126.3 | Momentive |
| GE9 H | 11.1 | 22.3 | |
| Byk A530 | 4.8 | 13.4 | Byk Chemie |
| *Mix the additives into the EPIKOTE resin before filler addition* | | | |
| Total | 79.1 | 162.0 | |
| FILLERS | Weight (parts) | Volume (parts) | Supplier |
| Sand 1-2 mm | 582.3 | 496.4 | SCR Sibelco |
| Sand 0.2-0.6 mm | 298.4 | 254.4 | SCR Sibelco |
| Total | 880.7 | 750.8 | |
| *Disperse into the base component using a concrete mixer* | | | |
| CURING AGENT COMPONENT | Weight (parts) | Volume (parts) | Supplier |
| EPIKURE F205 | 40.2 | 87.2 | Momentive |
| Total | 40.2 | 87.2 | |
| *Mix the curing agent well with the EPIKOTE resin base and Fillers before application* | | | |
| Total formulation | 1000.0 | 1000.0 | |

Example 7

Formulation for a water based self-leveling flooring The ingredients presented in the table below were mixed for the preparation of a waterbased self leveling flooring system.

| CURING AGENT COMPONENT (A) | Weight (parts) | Supplier | Comment |
|---|---|---|---|
| EPIKURE 8545-W-52 (HEW = 320 g/eq) | 164.00 | Momentive | |
| EPIKURE 3253 | 4.00 | Momentive | Accelerator |
| BYK 045 | 5.00 | BYK CHEMIE | defoamer |
| Antiterra 250 | 4.00 | BYK CHEMIE | Dispersing |
| Byketol WS | 5.00 | BYK CHEMIE | Wetting agent |
| Bentone EW (3% in water) | 20.00 | Elementis | Anti-settling |
| *Mix the additive into the EPIKURE curing agents before filler addition* | | | |
| Titanium dioxide 2056 | 50.00 | KronosTitan | |
| *Disperse the pigment for 10 minutes at 2000 rpm.* | | | |
| EWO-Heavy Spar | 195.00 | Sachtleben Chemie | Barium sulphate |
| Quartz powder W8 | 98.00 | Westdeutsche Quarzwerke | |
| *Disperse fillers at 2000 rpm for 10 minutes* | | | |
| Water | 55.00 | | |
| Sand 0.1-0.4 mm | 400.00 | Euroquarz | |
| Total component A | 1000.00 | | |
| RESIN COMPONENT (B) | | | |
| EPIKOTE 828LVEL | 81.00 | Momentive | |
| GE9 H | 19.00 | | |
| *Mix (B) into (A)* | | | |
| Total formulation A + B | 1081.00 | | |

Formulation characteristics

| | | |
|---|---|---|
| Fillers+Pigment / Binder ratio | 3.9 | by weight |
| PVC | 37.7 | % v/v |
| Density | 1.9 | g/ml |
| Water content | 12.5 | % m/m |

Example 8

The adducts of Glycidyl neononanoate, GE9 H or S and acrylic acid or methacrylic acid

The adducts of Glycidyl neononanoate GE9H with acrylic acid (ACE-adduct) and with methacrylic acid (MACE-adduct) are acrylic monomers that can be used to formulate hydroxyl functional (meth)acrylic polymers.

Compositions of the adducts intakes in parts by weight

| | Acrylic acid adduct | Meth acrylic acid adduct |
|---|---|---|
| Initial reactor charge | | |
| GE9H | 250 | 250 |
| Acrylic acid | 80 | |
| Methacrylic acid Radical Inhibitor | | 96.5 |
| 4-Methoxy phenol Catalyst | 0.463 | 0.463 |
| DABCO T9 (0.07 wt% on Glycidyl ester) | 0.175 | 0.175 |

- DABCO T9 and 4-Methoxy phenol (185 ppm calculated on glycidyl ester weight), are charged to the reactor.
- The reaction is performed under air flow (in order to recycle the radical inhibitor).
- The reactor charge is heated slowly under constant stirring to about 80°C, where an exothermic reaction starts, increasing the temperature to about 100°C.
- The temperature of 100°C is maintained, until an Epoxy Group Content below 30 meq/kg is reached. The reaction mixture is cooled to room temperature.

Example 9

Acrylic resins for high solids automotive refinish clearcoats

A glass reactor equipped with stirrer was flushed with nitrogen, and the initial reactor charge heated to 160°C. The monomer mixture including the initiator was then gradually added to the reactor via a pump over 4 hours at this temperature. Additional initiator was then fed into the reactor during another period of 1 hour at 160°C. Finally the polymer is cooled down to 135°C and diluted to a solids content of about 68% with xylene.

| Initial Reactor Charge | Weight % | in Reactor 1L (g) |
|---|---|---|
| GE 9H or GE9S | 28.2 | 169.1 |
| Xylene | 2.7 | 16.2 |
| Feeding materials | Weight % | in Reactor 1L (g) |
| Acrylic acid | 10 | 59.8 |
| Hydroxy ethyl methacrylate | 16.0 | 96.0 |
| Styrene | 30.0 | 180.0 |
| Methyl methacrylate | 15.8 | 95.0 |
| Di t-Amyl peroxide | 4.0 | 24.0 |
| Xylene | 8.3 | 49.8 |
| Post cooking | Weight % | in Reactor 1L (g) |
| Di t-Amyl peroxide | 1.0 | 6.0 |
| Xylene | 3.0 | 18.0 |
| Solvent adding at 130°C | Weight % | in Reactor 1L (g) |
| Xylene | 50.8 | 305.0 |
| | | |
| Final solids content | 61.8% | |
| Hydroxyl content | 4.12% | |

Example 10

Clear coats for automotive refinish

Solvents were blended to yield a thinner mixture of the following composition:

| Thinner | Weight % in solvent blend, theory |
|---|---|
| Toluene | 30.1% |
| ShellSolA | 34.9% |
| 2-ethoxyethyl acetate | 10.0% |
| n-Butyl acetate | 25.0% |
| **Total** | **100%** |

A clearcoat was then formulated with the following ingredients (parts by weight).

| Resin of example ex 9 | Desmodur N3390 | BYK 10wt% in ButAc | DBTDL 1wt% in ButAc | Thinner |
|---|---|---|---|---|
| 80.1 | 27.01 | 0.53 | 1.17 | 40.45 |

| **Clearcoat properties** | **GE9H** | **GE9S** |
|---|---|---|
| Volatile organic content | 480 g/l | 481 g/l |
| Initial viscosity | 54 cP | 54 cP |
| Dust free time | 12 minutes | 14.5 minutes |
| Koenig Hardness after 6 hours | 8.3 s | 7.1s |

Example 11

Acrylic resins for first finish automotive topcoats

GE9H based (28%) acrylic polymers for medium solids first-finish clear coats
A reactor for acrylic polyols is flushed with nitrogen and the initial reactor charge heated to 140°C. At this temperature the monomer mixture including the initiator is added over 4 hours to the reactor via a pump. Additional initiator is fed into the reactor during one hour, and then the mixture is kept at 140°C to complete the conversion in a post reaction. Finally the polymer is cooled down and diluted with butyl acetate to a solids content of about 60%.

**Paint Formulation**

Clear lacquers are formulated from the acrylic polymers by addition of Cymel 1158 (curing agent from CYTEC), and solvent to dilute to spray viscosity. The acidity of the polymer is sufficient to catalyse the curing process, therefore no additional acid catalyst is added. The lacquer is stirred well to obtain a homogeneous composition.

| | Intakes (parts by weight) |
|---|---|
| Initial reactor charge | |
| GE 9H | 164.40 |
| Xylene | 147.84 |
| Monomer mixture | |
| Acrylic acid | 53.11 |
| Butyl methacrylate | 76.88 |
| Butyl acrylate | 48.82 |
| Hydroxy-ethyl methacrylate | 27.20 |
| Styrene | 177.41 |
| Methyl methacrylate | 47.31 |
| Initiator | |
| Di-*tert*.-amyl peroxide (DTAP) | 8.87 |
| Post addition | |
| Di-*tert*.-amyl peroxide | 5.91 |
| Solvent (to dilute to 60% solids) | |
| Butyl acetate | 246.00 |
| Total | 1000.0 |

Clear lacquer formulations and properties of the polymers

| | Intakes (part by weight) |
|---|---|
| Ingredients | |

(continued)

| | Intakes (part by weight) |
|---|---|
| Acrylic polymer | 60.0 |
| Cymel 1158 | 8.8 |
| Butyl acetate (to application viscosity) | 24.1 |
| Properties | |
| Solids content [% m/m] | 45.3 |
| Density [g/ml] | 0.97 |
| VOC [g/l] | 531 |

Application and cure

The coatings are applied with a barcoater on Q-panels to achieve a dry film thickness of about 40μm. The systems are flashed-off at room temperature for 15 minutes, then baked at 140°C for 30 minutes. Tests on the cured systems are carried out after 1 day at 23°C.

## Claims

1. A process for synthesis of glycidyl ester from butene oligomers, comprising:

   (a) oligomerizing butenes or precursors of butene in presence of a catalyst **characterized in that** the olefin has a weight fraction of isobutene of at least 50 weight% on total alkene fraction of the mixture feed and that the subsequently mixture of neononanoic acid (C9 acids) derivative obtained by such a process is providing a mixture where the sum of the concentration of the blocked and of the highly branched isomers is at least 50%, preferably above 60%, most preferably above 75%, or that the olefin has a weight fraction of n-butene isomers of at least 50 weight% on total alkene fraction of the mixture feed, and the subsequently mixture of neononanoic acid (C9 acids) derivatives will provide a mixture where the sum of the concentration of the blocked and highly branched isomers is maximum 55%, preferably below 40% and most preferably below 30%,
   (b) converting butene oligomers of step (a) to carboxylic acids which are longer by one carbon atom, and
   (c) converting the carboxylic acids to the corresponding glycidyl esters.

2. A binder composition comprising the glycidyl esters obtained according to claim 1 reacted in resins such as an hydroxyl functional polyester, or an hydroxyl functional polyether, or an hydroxyl functional acrylic resins or a mixture thereof.

3. The use of the glycidyl esters obtained according to claim 1 in blend with epoxy resins as reactive diluent.

4. The use of the glycidyl esters obtained according to claim 1 in a reaction product with acrylic acid or methacrylic acid and the use thereof.

5. The binder of claim 2 in which the hydroxyl polyester is **characterized by** a calculated hydroxyl value above 100 mgKOH/g and an average molecular weight (MW) lower as 5 000.

6. The binder of claim 2 in which the hydroxyl polyether is **characterized by** a calculated hydroxyl value above 120 mgKOH/g and an average molecular weight (MW) lower as 4 500 DA.

7. The binder of claim 2 in which the hydroxyl functional acrylic resins is **characterized by** a calculated hydroxyl value between 50 and 180 mgKOH/g and an average molecular weight (MW) between 2 500 and 50 000 DA.

8. The binder of claims 5 to 7 in which the subsequently mixture of neononanoic (C9 acids) glycidyl derivative obtained by the process of claim 1 has an isomer composition wherein the sum of the concentration of the blocked and of the highly branched isomers is at least 50%, preferably above 60% and most preferably above 75% .

9. The binder of claims 5 to 7 in which the subsequently mixture of neononanoic (C9 acids) glycidyl derivative obtained

by the process of claim 1 has an isomer composition wherein the sum of the concentration of blocked and highly branched isomers is maximum 55%, preferably below 40% and most preferably below 30%.

## Patentansprüche

1. Verfahren zur Synthese eines Glycidylesters aus Butenoligomeren, enthaltend:

   (a) Oligomerisieren von Butenen oder Vorläufern von Buten in Gegenwart eines Katalysators, **dadurch gekennzeichnet, dass** das Olefin einen Gewichtsanteil an Isobuten von mindestens 50 Gew .-%, bezogen auf die gesamte Alkenfraktion des Gemisches aufweist, und dass das nachfolgende Gemisch aus Neononansäure (C9-Säuren)-derivat, das durch ein solches Verfahren erhalten wird, was eine Mischung liefert, bei der die Summe der Konzentration der blockierten und der hochverzweigten Isomere mindestens 50 %, vorzugsweise mehr als 60 %, am meisten bevorzugt mehr als 75 % beträgt, oder dass das Olefin einen Gewichtsanteil an n-Buten-Isomeren von mindestens 50 Gew.-%, bezogen auf die gesamte Alkenfraktion des Gemischs aufweist, und das nachfolgende Gemisch aus Neononansäure- (C9-Säuren) derivaten ein Gemisch ergibt, bei dem die Summe der Konzentration der Konzentration blockierter und hochverzweigter Isomere maximal 55 %, vorzugsweise unter 40 % und am meisten bevorzugt unter 30 % beträgt,
   (b) Umwandeln von Butenoligomeren von Schritt (a) in Carbonsäuren, die um ein Kohlenstoffatom länger sind, und
   (c) Überführen der Carbonsäuren in die entsprechenden Glycidylester.

2. Bindemittelzusammensetzung, enthaltend die nach Anspruch 1 erhaltenen Glycidylester, umgesetzt in Harzen wie einem hydroxyfunktionellen Polyester oder einem hydroxyfunktionellen Polyether oder hydroxyfunktionellen Acrylharzen oder einer Mischung davon.

3. Verwendung der nach Anspruch 1 erhaltenen Glycidylester im Gemisch mit Epoxidharzen als Reaktivverdünner.

4. Verwendung der nach Anspruch 1 erhaltenen Glycidylester in einem Umsetzungsprodukt mit Acrylsäure oder Methacrylsäure und deren Verwendung.

5. Bindemittel nach Anspruch 2, **dadurch gekennzeichnet, dass** der Hydroxypolyester einen berechneten Hydroxylwert von über 100 mg KOH / g und ein mittleres Molekulargewicht (MW) von weniger als 5 000 aufweist.

6. Bindemittel nach Anspruch 2, **dadurch gekennzeichnet, dass** der Hydroxypolyether einen berechneten Hydroxylwert von über 120 mg KOH / g und ein mittleres Molekulargewicht (MW) von weniger als 4 500 DA aufweist.

7. Bindemittel nach Anspruch 2, **dadurch gekennzeichnet, dass** die hydroxyfunktionellen Acrylharze einen berechneten Hydroxylwert zwischen 50 und 180 mg KOH / g und ein mittleres Molekulargewicht (MW) zwischen 2 500 und 50 000 DA aufweisen.

8. Bindemittel nach den Ansprüchen 5 bis 7, in dem das nachfolgend erhaltene Gemisch aus Neononansäure (C9-Säuren)-glycidylderivat erhalten nach dem Verfahren nach Anspruch 1, eine Isomerenzusammensetzung aufweist, wobei die Summe der Konzentration der blockierten und der hochverzweigten Isomere mindestens 50 %, vorzugsweise über 60 % und am meisten bevorzugt über 75 %, beträgt.

9. Bindemittel nach den Ansprüchen 5 bis 7, in dem das nachfolgend erhaltene Gemisch aus Neononansäure (C9-Säuren)-glycidylderivat erhalten nach dem Verfahren nach Anspruch 1, eine Isomerenzusammensetzung aufweist, wobei die Summe der Konzentration der blockierten und hochverzweigten Isomere maximal 55 %, vorzugsweise unter 40 % und am meisten bevorzugt unter 30 %, beträgt.

## Revendications

1. Un procédé de synthèse d’ester glycidylique à partir d’oligomères de butène, comprenant:

   (a) des butènes oligomériseurs ou des précurseurs de butène en présence d’un catalyseur **caractérisé en ce que** l’oléfine a une fraction pondérale en isobutène d’au moins 50% en poids de la fraction totale en alcène de

mélange d'alimentation, et que le mélange ultérieur de dérivés de néononaoïque acides (acides en C9) obtenu par un tel procédé fournit un mélange où la somme de la concentration des isomères bloqués et hautement ramifiés est d'au moins 50%, de préférence supérieure à 60%, le plus préférablement supérieure à 75% ou que l'oléfine a une fraction pondérale en isomères de n-butène d'au moins 50% en poids de la fraction totale d'alcène du mélange d'alimentation et le mélange ultérieur de dérivés de néononaoïque (acides C9) fournira un mélange où la somme de la concentration des isomères bloqués et hautement ramifiés est de 55% maximum, de préférence inférieur à 40% et le plus préférablement inférieur à 30%,

(b) conversion des oligomères de butène en acides carboxyliques plus longs d'un atome de carbone, et

(c) convertir les acides carboxyliques en les esters glycidyliques correspondants.

**2.** Une composition de liant comprenant les esters glycidyliques obtenus selon les revendication 1, mis à réagir dans des résines telles qu'un polyester à fonction hydroxyle ou un polyéther à fonction hydroxyle, ou des résines acryliques à fonction hydroxyle ou un mélange de celles-ci.

**3.** L'utilisation des esters glycidyliques obtenus selon la revendications 1 en mélange avec des résines époxydes en tant que diluant réactif.

**4.** L'utilisation des esters glycidyliques obtenus selon la revendication 1 dans un produit de réaction avec de l'acide acrylique ou de l'acide méthacrylique et leur utilisation.

**5.** Le liant selon la revendication 2, dans lequel le polyester hydroxyle est **caractérisé par** un indice d'hydroxyle calculé supérieur à 100 mg de KOH / g et un poids moléculaire moyen (MW) inférieur à 5 000 DA.

**6.** Le liant selon la revendication 2 dans lequel l'hydroxypolyéther est **caractérisé par** un indice d'hydroxyle calculé supérieur à 120 mg de KOH / g et un poids moléculaire moyen (MW) inférieur à 4 500 DA.

**7.** Le liant selon la revendication 2 dans lequel les résines acryliques à fonction hydroxyle sont **caractérisées par** un indice d'hydroxyle calculé entre 50 et 180 mg de KOH / g et un poids moléculaire moyen (MW) entre 2 500 et 50 000 DA.

**8.** Le liant selon les revendications 5 à 7, dans lequel le mélange subséquent de dérivé glycidyle de néononaoïque (acides C9) obtenu par le procédé de la revendication 1 a une composition isomère dans laquelle la somme de la concentration des isomères bloqués et hautement ramifiés est au moins 50%, de préférence supérieur à 60% et le plus préférablement supérieur à 75%

**9.** Le liant selon les revendications 5 à 7, dans lequel le mélange subséquent de dérivé glycidyle de néononaoïque (acides C9) obtenu par le procédé de la revendication 1 a une composition isomère dans laquelle la somme de la concentration en isomères bloqués et hautement ramifiés est maximale 55%, de préférence inférieur à 40% et le plus préférablement inférieur à 30%.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2831877 A
- US 2876241 A
- US 3053869 A
- US 2967873 A
- US 3061621 A
- EP 1033360 A1
- EP 1033360 A
- EP 1281700 A
- EP 0996657 A
- EP 2010003334 W
- US 6433217 B


### Non-patent literature cited in the description


- **H VAN HOORN ; G C VEGTER.** Dynamic modulus measurements as a tool in the development of paint base materials FATIPEC. *Euro Continental Congress,* 1968, vol. 9, 51-60
- *Rheol. Acta,* 1971, vol. 10, 208-212